# EUROPEAN PATENT SPECIFICATION

(11) **EP 1 355 676 B1**
(45) Date of publication and mention of the grant of the patent: **05.10.2011**
(21) Application number: 01979641.6
(22) Date of filing: 11.10.2001
(51) Int. Cl.: A61K 49/00, A61K 47/48, G01N 33/68

(54) **METHOD OF PROTECTING THERAPEUTIC COMPOSITIONS FROM HOST-MEDIATED INACTIVATION**
METHODE ZUM SCHUTZ THERAPEUTISCHER ZUSAMMENSETZUNGEN VON HOSTVERMITTELTER INAKTIVIERUNG
METHODE DE PROTECTION DE COMPOSITIONS THERAPEUTIQUES CONTRE L'INACTIVATION INDUITE PAR UN HOTE

(30) Priority: 12.10.2000 US 239268 P
(43) Date of publication of application: 29.10.2003
(60) Divisional application: 10184001.5
(73) Proprietor: UNIVERSITY OF SOUTH CAROLINA, Columbia South Carolina 29208 (US)
(72) Inventor: ROBERTS, Joseph, Columbia, SC 29223 (US); SETHURAMAN, Natarajan, Columbia, SC 29210 (US)
(74) Representative: Schouboe, Anne
(86) International application number: PCT/US2001/031599
(87) International publication number: WO 2002/031498

(56) References cited:
- US-A- 5 880 255
- DATABASE MEDLINE [Online] US NATIONAL LIBRARY OF MEDICINE (NLM), BETHESDA, MD, US; ABUCHOWSKI A ET AL: "Immunosuppressive properties and circulating life of Achromobacter glutaminase - asparaginase covalently attached to polyethylene glycol in man." retrieved from STN Database accession no. 82048507 XP002225260 & CANCER TREATMENT REPORTS, (1981 NOV-DEC) 65 (11-12) 1077-81. ,
- FUERTGES F ET AL: "THE CLINICAL EFFICACY OF POLY(ETHYLENE CLYCOL)-MODIFIED PROTEINS" JOURNAL OF CONTROLLED RELEASE, ELSEVIER SCIENCE PUBLISHERS B.V. AMSTERDAM, NL, vol. 12, no. 1 - 3, 1990, pages 139-148, XP000113380 ISSN: 0168-3659
- HOLCENBERG, JOHN S. ET AL: "Biologic and physical properties of succinylated and glycosylated Acinetobacter glutaminase - asparaginase" J. BIOL. CHEM. (1975), 250(11), 4165-70 , XP001131946
- KAFKEWITZ D ET AL: "Enzyme-induced asparagine and glutamine depletion and immune system function." AMERICAN JOURNAL OF CLINICAL NUTRITION, (1983 JUN) 37 (6) 1025-30. REF: 36 , XP001127489

## Description

### BACKGROUND OF THE INVENTION

### FIELD OF THE INVENTION

The present invention relates to a method for determining the modification conditions that protect therapeutic compositions from host-mediated inactivation. The present invention also relates to modified therapeutic agents that are shielded from host-mediated inactivation.

### BACKGROUND OF THE INVENTION

When a host encounters a foreign agent in its circulation, the host's immune system may initiate an immune response. This response includes the production of agent-inactivating antibodies that also enable the reticuloendothelial system to clear the agent from circulation. Thus, the therapeutic life of an administered non-human agent is often limited by the host's immune system. Furthermore, the problem of host-mediated defense limits the usefulness of human proteins that either are not generally found in circulation or are produced in heterologous systems, using recombinant DNA technology (Nucci, ML, Short, R and Abuchowski, A., 1991. Advanced Drug Delivery Reviews, 6:133-151).

One strategy for protecting these agents is to covalently modify them with agents like carbohydrates or biocompatible polymers, like Polyethylene Glycol ("PEG"). The "PEGylation" of proteins, for example, has been used to shield such proteins from the immune system (Nucci, ML, Short, R and Abuchowski, A., 1991. Advanced Drug Delivery Reviews, 6:133-151; Zalipsky, S. 1995. Advanced Drug Delivery Reviews, 16:157-182). PEGylation increases the stability of proteins (Nucci, ML, Short, R and Abuchowski, A., 1991. Advanced Drug Delivery Reviews, 6:133-151) and also greatly reduces their antigenicity (measured by the extent of reaction of antibodies raised against unmodified protein to PEGylated protein) and immunogenicity (measured by the extent of antibody formation against PEGylated protein in treated animals), as measured by *in vitro* immunological techniques (Abuchowski, A., Van, Es, T., Palczuk, NC and Davids, FF., 1977. Journal ofBiol. Chem., 11:3578-3581).

One drawback of protein modification by PEGylation, however, is that PEGylation often reduces the biological activity of the agent. The extent of reduction varies with the type of activated PEG used and the number of Polyethylene Glycol moieties attached to the agent. The biological activity also is affected by the extent to which an agent is modified with a particular PEG.

Currently, the choice of the activated PEG used and the extent of PEGylation are based on the two main criteria: acceptable loss of therapeutic activity (Francis, GE, Delgardo, C, Fisher, D, Malik, F and Agrawal, AK., 1996. J. Drug Targeting, 3:321-340), and the reduction of antigenicity and immunogenicity of the agent. However, the assessment of the PEG modification of a given agent, according to these criteria, often leads to an arbitrary result. In one such arbitrary example, *Pseudomonas 7A* Glutaminase-Asparaginase (PGA) was modified by as much as 74%, using SC-PEG 5000, resulting in less than 20% loss in activity (U.S. Pat. No. 5,808,096; 5,612,460; 5,324,844; 5,122,614).

Chinol M. et al.; British Journal of Cancer, vol 78, no. 2, 1 July 1998, pages 189-197 relates to a method of assaying the effects on immunogenicity, pharmacokinetics and biodistribution of modification to streptavidin, however this anti-avidin reactivity does not constitute biological activity as defined in the present invention.

Deckert M. et al.; International Journal of Cancer, vol 87, 2000, pages 382-390 relates to a method of assaying immunogenicity of PEGylated humanized A33 antibodies, however the cited data are not indicative of an evaluation of blood samples for the binding activities of administered PEGylated antibodies.

The existing criteria (antigenicity, immunogenicity and acceptable loss of bioactivity) for determining the activated PEG used and the extent of PEGylation are insufficient for ascertaining the modification for an agent in instances where such agents are administered to a patient over a prolonged period of time. This is true, because none of the foregoing criteria take into consideration the effect of the host's response on the agent's biological activity *after* the PEGylated agent is administered to the host. As a result, reliance on only the aforementioned criteria will produce an agent that is not optimally protected from the host's immune system, or otherwise from *in situ* inactivation.

Accordingly, there is a great and present need for a strategy that would enable the skilled artisan to determine the extent ofPEGylation for a given agent that is administered to a patient during prolonged periods of therapy. The inventors have found that when a novel, physiologically relevant optimization scheme is applied and the modifying agent is a polyethylene glycol, these modification ("PEGylation") ranges are not commensurate with the highest suitable PEGylation range as determined by employing an *in vitro* assays that monitors loss of therapeutic activity, antigenicity and immunogenicity. Currently, these *in vitro* methods of determining suitable PEGylation ranges are the only existing method as described in the art. The inventors' discovery suggests that over-PEGylation of an agent, as well as over-modification of an agent with any modifying agent, can disrupt the secondary and/or tertiary structure of the agent, thus exposing new antigenic determinants to the immune system. It is understood that this observation applies equally to other bio-compatible polymers or agents used to increase the useful circulating half-life of therapeutic agents.

### SUMMARY OF THE INVENTION

A method for determining the type of biocompatible polymer, the extent of modification, and the conditions for modification of a therapeutic agent with a biocompatible polymer to prevent host-mediated inactivation of said therapeutic agent when covalently modified by said biocompatible polymer, comprising:
(a) assaying a biological activity of a first modified therapeutic agent after said first modified therapeutic agent has been administered to a **non-human** subject, wherein said first modified therapeutic agent is covalently modified with a biocompatible polymer;
(b) assaying the biological activity of said first modified therapeutic agent after at least one booster dose of said first modified therapeutic agent has been administered to said **non-human** subject;
(c) assaying the biological activity of a second modified therapeutic agent after said second modified therapeutic agent has been administered to a **non-human** subject, wherein said second modified therapeutic agent is covalently modified with a biocompatible polymer and wherein at least one condition selected from the group consisting of the type of biocompatible polymer, the extent of modification, and the conditions for modification differs from the conditions of said first modified therapeutic agent;
(d) assaying the biological activity of said second modified therapeutic agent after at least one booster dose of said second modified therapeutic agent has been administered to said **non-human** subject; and
(e) comparing the biological activity of said first modified therapeutic agent with the biological activity of said second modified therapeutic agent to select the type of biocompatible polymer, the extent of modification, and the conditions for modification that prevent host-mediated inactivation of said therapeutic agent when covalently modified by said biocompatible polymer,
wherein said therapeutic agent comprises an enzyme and said biological activity comprises an enzyme catalyzing a reactiont.

The present invention relates to a modified therapeutic composition suitable for therapeutic administration to a subject over a prolonged period comprising a glutaminase-asparaginase (GA) and a polyethylene glycol (PEG), wherein the GA is PEGylated between about 21% and 49%, and wherein the PEG is SC-PEG 5000.

The present invention also relates to a modified therapeutic composition suitable for therapeutic administration to a subject over a prolonged period comprising a GA and a PEG, wherein the GA is PEGylated to between about 25% and 58%, and wherein the PEG is SBA-PEG 5000.

The present invention further relates to a modified therapeutic composition suitable for therapeutic administration to a subject over a prolonged period comprising a GA and a PEG, wherein the GA is PEGylated to between about 45% and 65%, and wherein the PEG is ALD-PEG 2000.

The present invention further relates to a modified therapeutic composition suitable for therapeutic administration to a subject over a prolonged period comprising a GA and a PEG, wherein the GA is PEGylated to between about 25% and 65%, and wherein the PEG is SPA-PEG 5000.

### BRIEF DESCRIPTION OF THE DRAWINGS

Figure 1 is a graph describing animal experiments with PGA modified with SS-PEG.
Figure 2 is a graph describing animal experiments with PGA modified with BTC-PEG.
Figure 3 is a graph describing animal experiments with PGA modified with ALD-PEG.
Figure 4 is a graph describing animal experiments with PGA modified with SC-PEG.
Figure 5 is a graph describing animal experiments with PGA modified with SBA-PEG.
Figure 6 is a graph illustrating MALDI analysis of PGA Modified with SPA-PEG.
Figure 7 is a graph describing animal experiments with PGA modified with SPA-PEG.
Figure 8 is a graph describing the protective capabilities of compounds during PGA lyophilization.
Figure 9 is a graph describing animal experiments of Formulation Development with PGA modified with SPA-PEG.

### DETAILED DESCRIPTION OF THE PREFERRED EMBODIMENTS

A method for determining the type of biocompatible polymer, the extent of modification, and the conditions for modification of a therapeutic agent with a biocompatible polymer to prevent host-mediated inactivation of said therapeutic agent when covalently modified by said biocompatible polymer, comprising:
(a) assaying a biological activity of a first modified therapeutic agent after said first modified therapeutic agent has been administered to a **non-human** subject, wherein said first modified therapeutic agent is covalently modified with a biocompatible polymer;
(b) assaying the biological activity of said first modified therapeutic agent after at least one booster dose of said first modified therapeutic agent has been administered to said **non-human** subject;
(c) assaying the biological activity of a second modified therapeutic agent after said second modified therapeutic agent has been administered to a **non-human** subject, wherein said second modified therapeutic agent is covalently modified with a biocompatible polymer and wherein at least one condition selected from the group consisting of the type ofbiocompatible polymer, the extent of modification, and the conditions for modification differs from the conditions of said first modified therapeutic agent;
(d) assaying the biological activity of said second modified therapeutic agent after at least one booster dose of said second modified therapeutic agent has been administered to said **non-human** subject; and
(e) comparing the biological activity of said first modified therapeutic agent with the biological activity of said second modified therapeutic agent to select the type of biocompatible polymer, the extent of modification, and the conditions for modification that prevent host-mediated inactivation of said therapeutic agent when covalently modified by said biocompatible polymer,
wherein said therapeutic agent comprises an enzyme and said biological activity comprises an enzyme catalyzing a reactionPrevention of host-mediated inactivation, as used herein, means that the subject's normal self-defense response, or the effects thereof, to the therapeutic agent is diminished. The self-defense response can include the production of agent-inactivating antibodies that enable the reticuloendothelial system to clear the agent from circulation, as well as inducement of detoxifying mechanisms that serve to convert the therapeutic agent to a therapeutically inactive compound. Because of the host's self defense mechanisms, the therapeutic life of an administered non-human agent is often limited. Thus the invention provides a direct *in vivo* functional method of ascertaining modification conditions of a therapeutic agent that allow the agent to evade the host's self-defense mechanisms and consequently extend the effective therapeutic life of the therapeutic agent in the host. Prevention can mean a complete or partial reduction in the efficacy of the host's normal inactivating mechanisms towards the modified therapeutic agent, such that the therapeutic life of the agent is augmented beyond the unmodified or previously modified therapeutic agents.

In the method of the current invention, the first and additional therapeutic agent are the same active ingredients or structurally-related analogs, but they have been modified differently in at least one aspect. In one embodiment, the therapeutic agents are identical, and the modifying agents are identical, however the amount or extent of modifying agent linked to the therapeutic agent is different. In another embodiment, the therapeutic agents are identical, but the modifying agents are not. As used herein here, "additional" can mean one or more, thus the methods described herein include comparing one or more modified therapeutic agents to the first modified therapeutic.

Modification conditions refers to parameters of the type of modifying agent linked or joined to the therapeutic agent as well as the extent and conditions of modification. As understood in the context of the present invention, the extent or degree or amount of modification of the therapeutic agent is used to mean the number of residues of an amino-acid of a therapeutic agent that are modified by the modifying agent divided by the total number of amino acids available for modification, expressed as a percent. For example, an agent that has been "PEGylated" 20% (meaning the agent has been modified by linking it with a polyethylene glycol (PEG)) means that PEG has been joined or linked to 20% of the lysine residues of the therapeutic agent. Other parameters of modification conditions include the type of modifying agent used (e.g. bio-compatible polymers), the buffer, pH, pressure and temperature under which the modification takes place, the conformational state of the therapeutic agent, the type of therapeutic agent used (e.g. a parent compound versus any of its structurally-related analogs), and the physical state of the modifying agent or therapeutic agent, including the liquid, solid and gas states, attached to matrix, bound to substrate of product, a solid that has been lyophilized, a liquid that been frozen or a gas that has been condensed.

Percent modification of a polypeptide, for example, can be determined by one of a variety of conventional techniques. According to one embodiment, the modified polypeptide is assayed with an assay that detects a particular free amino acid of a polypeptide, wherein the free amino acid represents the type of amino acid with which the selected modifying agent reacts. For example, the fluorescamine reacts with unmodified lysine groups, yielding a fluorescent derivative. An assay comprising fluorescamine would be a suitable assay to employ, for example, under conditions wherein the polypeptide of interest is reacted with a modifying agent that has an affinity for lysine. By comparing the deviation in production of fluorescent derivative between a polypeptide that is not modified and a polypeptide that is modified, a skilled artisan will be able to ascertain the extent of modification associated with the particular polypeptide.

The extent of PEGylation of mercapto or sulfhydryl group specific PEG can be determined by a spectrophotometric method. An exemplary method quantitates the available cysteine sulfhydryl groups, as indicated by reacting these functional groups with 2,2'-dipyridyl disulfide to form 2-thiopyridone. At a pH of 7.2, 2-thiopyridone adsorbs at 343 nm, where e = 7060. In another effective approach, Ellman's reagent [5,5'-dithiobis(2-nitrobenzoic acid)] is substituted for 2,2'-dipyridyl disulfide. In another embodiment, the extent of modification of a polypeptide can be determined by Matrix-assisted-laser-desorption-ionization mass spectrometry (MALDI). MALDI provides the mass of the unfragmented, singly-charged molecular ion of macromolecules up to about 100,000 daltons and, thus, greatly assists determination of the mass of the molecular ions of PEG conjugates, such as PEGylated polypeptides. In addition, the composition of PEGylation reaction products that contain different numbers of PEGs ("one-mer", "two-mer", etc.) can be established by using MALDI technology. Accordingly, the invention contemplates a variety of techniques for deducing the extent, or percent, to which an agent is modified.

The percent modification of a particular agent or other suitable therapeutic composition, as the starting material, can vary without departing from the scope of the invention. For example, an agent initially can be modified to between about 25% and about 75%, then administered to a subject according to the methods disclosed herein.

According to one embodiment of the invention, once a particular therapeutic agent or composition is selected, this agent or composition may be subjected to a modification strategy, such as PEGylation, to determine the modification conditions for the agent or composition for administration to a subject. Of course, the modification conditions for a given agent or therapeutic composition will vary, according to the type of modifying agent employed and the properties of the agent or composition. In one embodiment, the selected modifying agent is reacted with a particular agent before the modified agent is administered to a subject. In a preferred embodiment, the agent or therapeutic composition is first purified and dissolved in a suspension of fluid, for example a standard buffer solution, before being reacted with the modifying agent. To this end, separate aliquots of the agent or composition are obtained. Thereafter, if the skilled artisan is attempting to ascertain a degree of modification for the same modifying agent, for example, a concentration of the selected PEG is added to each aliquot, wherein the concentration of the modifying agent is different for each aliquot. The agent or composition and the modifying agent are reacted such that a portion of the concentration of modifying agent or composition covalently attaches to the agent, thereby modifying the agent or composition. Accordingly, each aliquot should contain an agent or composition modified to a percentage that is distinct from the percentage modification associated with the other aliquots of modified agent or composition. When the therapeutic agent or composition is a vector containing a nucleic acid, the modifying agent may be attached, for example, to a lysine amino group or other functional group(s) of surface antigenic determinants.

Suitable therapeutic agents, according to the invention include amino acids, polypeptides, nucleic acids, carbohydrates, lipids, or small molecules such as a drug. Furthermore, a therapeutic agent is an agent that is capable of conferring a therapeutic benefit to a subject or patient. The terms agent or compound are used interchangeably herein. As the invention contemplates, the phrase "therapeutic benefit" is intended to mean a physiological or cellular response to an agent that alleviates, reduces and removes the symptoms of a condition or disease in patients in need of treatment of the condition or disease. Therapeutic benefit can also mean a physiological or cellular response to an agent where the patient is "cured," or the causal agents or factors of the condition from which the patient is suffering are removed, destroyed or corrected. In one embodiment, the invention contemplates the use of a polypeptide as a therapeutic agent. In a further embodiment, this polypeptide comprises a monomeric molecular weight of about 300 to about 300,000 daltons.

In one embodiment, the therapeutic agents or compositions can be used to treat cancer in subjects in need of treatment thereof. There are numerous types of cancers that can be treated according to the invention, including prostate and ovarian cancer, and glioblastomas. Other types of cancers that may be treated include: chronic and acute leukemia, cancer of the bone, brain, breast cartilage, cervix, esophagus, kidney, larynx, liver, lung, pancreas, and uterus. In addition, the agents or compositions of the invention may be used to combat Hodgkin's Disease, lymphoma, melanoma, multiple myeloma, colo-rectal, and testicular cancer.

In another embodiment, the therapeutic agents or compositions can be used to treat viral infections in subjects in need of treatment thereof. Accordingly, the modified agents or compositions of the invention can be effective against these three main groups of viruses. Specific viruses that can be treated according to the invention include human Respiratory Syncytial Virus (RSV), Herpes Simplex Virus (HSV) and Human Immunodeficiency Virus (HIV).

Other treatable viruses include the following closely related viruses. Respiratory syncytial virus belongs to the family Paramyxoviridae. The other human infectious viruses belonging to the family Paramyxoviridae include: Parainfluenza 1, 2, 3, 4 viruses which cause upper respiratory disease, bronchitis/bronchiolitis, pneumonia; mumps virus, and measles virus. The family Paramyxoviridae is very closely related to Rhabdoviridae and Filoviridae because the viruses belonging to these families contain a single-stranded RNA (negative sense) genome which is non-segmented and enveloped. Human infectious viruses belonging to Rhabdoviridae are vesicular stomatitis-Indiana, New Jersey, cocal viruses, chandipura virus, Piry virus, Isfahan virus, rabies virus, Mokola virus, and Duvenhage virus. Human infectious viruses belonging to the family Filoviridae include Marburg and Ebola viruses. More broadly, Respiratory Syncytial Virus is an RNA virus.

Other RNA viruses that cause human infections include the following: polioviruses 1, 2, and 3; coxsackieviruses B1-B6; human echoviruses 1-9, 11-27, and 29-34; human enteroviruses 1-113; Norwalk virus and similar viruses that belong to the family Caliciviruses that cause gastroenteritis in humans; eastern equine encephalitis virus; western equine encephalitis virus; Venezualan equine encephalitis virus; chikungunya virus; O'nyong-nyong virus; Ross River virus; Mayarovirus; rubella virus; yellow fever virus; dengue viruses; Western Nile virus; St. Louis encephalitis virus; Japanese encephalitis virus; Murray Valley encephalitis virus; Rocio virus; tick-borne encephalitis viruses; human coronaviruses 229-E and OC43; upper respiratory tract infection, probably pneumonia and possibly gastroenteritis; influenza A, B, and C viruses; Bunyamwera virus; Bwamba virus; Oriboca virus; Oropouche virus; Gwama virus; California encephalitis virus; LaCrosse virus; Tahyna virus; Sandfly fever-Naples virus; Crimean-Congo hemorrhagic fever virus; Hantaan virus (Korean hemorrhagic fever, hemorrhagic fever with renal syndrome, nephropalthia epidemica); lymphocytic choriomeningitis (LCM) virus; Lassa virus; Machupa virus (Bolivian hemorrhagic fever); Junin virus (Argentine hemorrhagic fever); reovirus 1, 2, and 3; Orungo virus (febrile illness in Nigeria and Uganda); Kemerovo virus (febrile illness in Russia and Egypt); human rotaviruses, Colorado tick fever virus.

Rauscher Murine Leukemia virus belongs to the family Retroviridae. Viruses that belong to this family have a single-stranded (positive sense), non-segmented enveloped genome, but they involve a DNA step in replication. Human infectious viruses belonging to this family include type C oncoviruses such as human T-lymphotropic virus 1 (HTLV-I, adult T-cell leukemia) and human T-lymphotropic virus 2 (HTLV-II, possibly associated with hairy-cell leukemia), human immunodeficiency viruses 1 and 2 (HIV 1 and HIV 2) that cause acquired immunodeficiency syndrome (AIDS) and other viruses, related to HIV 1 and HIV 2, which cause AIDS-like disease.

Herpes Simplex Virus belongs to the family Herpesviridae. Viruses belonging to the family Herpesviridae have a double-stranded enveloped genome, a property that they share with viruses belonging to the families Poxviridae and Iridoviridae. Human infectious viruses belonging to the family Herpesviridae include Herpes Simplex Viruses 1 and 2, cercopilthecine, herpesvirus 1 (B-virus), varicella-Zoster virus, human cytomegalovirus, EB virus, and human herpesvirus 6. Human infectious viruses belonging to Poxviridae include variola virus (smallpox, alastrim), vaccinia virus, monkeypox virus, cowpox virus, orf virus (contagious pustular dermatitis), pseudo-cowpox (milker's nodule) virus, yabapox virus, tanapox virus, and molluscum contagiosum virus. More broadly, Herpes Simplex Virus is a DNA virus and other human infectious viruses in this category are hepatitis B virus; human parvovirus B-19, parvovirus RA-1, and other parvoviruses that cause gastroenteritis; human papillomaviruses (HPV) 1-48); polyomaviruses such as JC, SV40 and BK; and Adenoviruses such as Mastadenovirus h1-h49.

As stated previously, the therapeutic agent can be a polypeptide. The terms "protein," "peptide" and "polypeptide" are used interchangeably. Examples of suitable polypeptides include, but are not limited to, oxidoreductases, such as oxygen oxidoreductase (1.7.3.3; "uricase"), hydrogen-peroxide oxidoreductase (1.11.1.6; "catalase"), and oxygen oxidoreductase (20-.beta.-hydroxylating) (1.14.1.9; "Cholesterol 20-hydroxylase"); transferases, such as glucuronyltransferase (2.4.1.17; "UDP glucuronyltransferase"), and α-D-Galactose-1-phosphate uridylyltransferase (2.7.7.12); hydrolases, such as Mucopeptide N-acetylmuramylhydrolase (3.2.1.17; lysozyme), trypsin (3.4.4.4), and L-Asparagine aminohydrolase (3.5.1.1; "Asparaginase"); lyases, such as Fructose-1,6-diphosphate D-glyceraldehyde-3-phosphate-lyase (4.1.2.12: "aldolase"); Isomerases, such as D-Xylose ketol-isomerase (5.3.1.5, xylose isomerase); and Ligases, such as L-aspartate ligase (AMP) (6.3.4.5). Several proteins, polypeptides, peptides and enzymes have been shown to have therapeutic activity.

Among other polypeptides contemplated by the invention are therapeutically useful enzymes (natural and recombinant), such as: carbohydrate-specific enzymes, proteolytic enzymes, oxidoreductases, transferases, hydrolases, lyases, isomerases and ligases. Examples of therapeutic enzymes include asparaginase, glutaminase-asparaginase, such as *Pseudomonas 7A* glutaminase-asparaginase ("PGA"), arginase, arginine deiminase, arginine decarboxylase, lysine decarboxylase, histidine ammonia lyase, adenosine deaminase, superoxide dismutase, DNase, endotoxinases, catalases, chymotrypsin, lipases, uricases, adenosine diphosphatase, tyrosinases, bilirubin oxidase, glucose oxidase, glucodases, galactosidases, glucocerebrosidases, glucouronidases, thrombolytic agents Activase, (alteplase), Streptase (streptokinase), Abbokinase (urokinase) and Eminase (anistreplase), clotting factors VII, VIII, and IX, anticoagulants Refludan, Antithrombin and Integrillin, α-Antitrypsin, ureate oxidase, Indolyl-3-alkene α-hydroxylase (IAH), methioninase, neuraminidase, phenyalanine ammonia lyase, and lipases.

Other polypeptides include: hormones and growth factors, such as Insulin, Erythropoietin and hormones with Erythropoietin like activities, growth hormones, growth hormone releasing factor, Insulin-like growth factor, platelet-derived growth factor (PDGF), phospholipase-activating protein (PLAP), transforming growth factor-beta (TGF-β), tissue growth factors α and β epithelial growth factor, fibroblast growth hormone, keratinocyte growth hormone, leptin, nerve growth factors, bone morphogenic factors, stem cell factors, fertility hormones, human parathyroid hormone, thyroid stimulating hormone, hypothalamic releasing factors, antidiuretic hormone, prolactin, chorionic gonadotropin, follicle-stimulating hormone, corticotropin-releasing factor, vascular endothelial growth factor, amlin, insulinotropin, glucagons, endostatin, angiostatin, somatomedins, pigmentary hormones; enzymes such as asparaginase, glutaminase-asparaginase; cytokines like interferon-α, interferon-β, interferon-γ, tumor necrosis factor-α (TNF-α), tumor necrosis factor receptor, granulocyte-colony stimulating factor, granulocyte macrophage-colony stimulating factor, interleukins 1 through 15, myeloid proginator inhibitory factor-1, interleukin receptor antagonist (IL-1ra), TNF-receptor fusion protein, T-cell inhibitory protein, hemoglobin, serum proteins such as Factors VII, VIII and IX, immunoglobulins (human or other mammalian) such as IgG, IgE, IgM, IgA, IgD and fragments thereof (including Fab's and scFv's), and plant proteins, for example, lectins and ricins.

The invention also contemplates, in one embodiment, the use of agents that exhibit anti-microbial activities, such as anti-fungal and anti-bacterial activity. Examples of parent anti-microbial polypeptides, according to the invention, include: fungicidally active polypeptides that are derived from the mold genus Curvularia, as described in, for example, WO 94/01459; anti-bacterial polypeptides, such as those described in EP 403.458; anti-microbial proteins isolated from the Mirabilis seed, described, e.g., in WO 92/15691; anti-bacterial polypeptides isolated from an extract of pig small intestine, as described in WO 92/22578; polypeptides with yeast lethal action, as accumulated by yeast of *Hansenula* spp., described, for example, in JP-60130599; *Phytolacca insularis* antiviral protein, which can be used as an anti-microbial described in U.S. Patent No. 5,348,865; and bacteriolytic enzyme preparations derived from *Nocardiopsis dassonvillei*, as described, *e.g.*, in U.S. Pat. No. 5,354,68 1. Further examples of other anti-microbial polypeptides, which also are contemplated by the invention, include: maganinin, protegrin, defensin, pseudomycin, mutanolysin and N-acetylneuramidase.

The invention further contemplates the use of naturally available polypeptides, as well as polypeptides produced using recombinant DNA technology. Such polypeptides include non-glycosylated polypeptides and polypeptides that are not modified by post-translational modification (e.g. phosphorylation, glycosylation, etc.). The invention also contemplates the use of partial peptides having bio-activity, as well as fusion proteins that connect two or more functional groups.

In another embodiment, the invention includes the use of additional therapeutic agents, for example, nucleic acids. Nucleic acids can be administered to subjects directly, or as part of a plasmid or viral vector. The administration thereof to a subject should be in accordance with the methods described herein. Suitable vectors, according to the invention, include vectors derived from retroviruses, adeno-viruses, adeno-associated viruses, herpes virus, lentivirus, or any other virus useful in gene therapy. A vector is "useful" in gene therapy if the vector is capable of delivering a gene to a host cell. According to one embodiment, the vector comprises a nucleic acid sequence from which a polypeptide of interest subsequently is transcribed. In another embodiment, the nucleic acids are used to treat viral infections, such as, for example, human immunodeficiency virus (HIV) in subjects in need of treatment thereof. In an additional embodiment, the nucleic acids are used to treat cancer in subjects in need of treatment thereof.

The methods as provided in the current invention involve ascertaining the modification conditions of a therapeutic agent, by assessing the biological activity of the modified agent, after administration to an animal or subject. By "biological activity" is meant a cellular or physiological response or reaction that that agent causes, either directly or indirectly. The biological activity can be assessed *in vivo*, *in vitro* or *in situ*. Examples of biological activity include an enzyme catalyzing a reaction, a molecule binding a receptor or antibody, mediating a receptor-mediated response such as ion influx/efflux or generation of second messengers, antagonizing or blocking a receptor-mediated response, induction of apoptosis and release or uptake of a neurotransmitter or hormone. The biological activity is not necessarily the same activity as the therapeutic benefit that the agent bestows upon the subject. For example, a skilled artisan could measure the ability of a modified therapeutic to bind to a receptor, *in vitro* or *in situ*, to demonstrate that the therapeutic agent confers a therapeutic benefit. However, the type of assay to be employed will vary, according to the type of therapeutic that is being administered. A suitable assay is any assay that is able to detect the biological activity of the therapeutic. For example, if the administered agent possesses glutaminase activity, then a suitable assay in this instance would be one that is able to detect any such glutaminase activity (Roberts, J., 1976. Journal ofBiological Chemistry, 251:2119-2123). If the administered therapeutic is a vector which comprises a nucleic acid sequence, for example, capable of transcribing a polypeptide that possesses a biological activity, then a suitable assay would be an assay capable of detecting the biological activity of the transcribed polypeptide. If the administered therapeutic does not have a simple assay to measure biological activity in the serum, immunological assays can be used to quantify the amount of the therapeutic composition in the serum.

Subsequent to assaying this initial dose, the modified therapeutic is administered to the subject in one or more booster doses. As used herein, a "booster dose" is a dose that tends to mimic the amount of therapeutic agent that a subject would receive, according to a conventional therapeutic dosage regimen. A booster dose can be more than, less than, or equal to the initial dose, as described above. In a preferred embodiment, the booster dose is of lesser concentration than the initial dose. A "therapeutic dosing regimen" refers to an amount of a therapeutic agent that is sufficient to bring about a therapeutic benefit to a subject.

As used herein, the terms subject, patient and animal are used interchangeably. Suitable subjects, according to the invention, include non-human mammals, in particular rodents, canines, cats, horses, cattle, and non-human primates.

As used herein, the term "modified therapeutic agent" means a therapeutic agent wherein a modifying agent has been linked or joined to the therapeutic agent. By modifying agent, it is meant an agent or compound that reduces the immunogenic or antigenic response of the subject towards the therapeutic agent, compared to the immunogenic or antigenic response of the subject towards the same therapeutic agent that has not been modified. Examples of modifying agents include bio-compatible polymers. For example, a polymer such as polyethylene glycol (PEG) can be appended to a drug or polypeptide and this will reduce the subject's immune response to the polypeptide while still conferring a benefit to the subject. Without the presence of the appended PEG, the drug or polypeptide may be neutralized by the subject's immune system, resulting in a partial or total loss of any biological activity that the drug or polypeptide can bestow upon the subject.

Suitable bio-compatible polymers, according to the present invention, include: homopolymers, copolymers and block copolymers of monoalkyl-capped polyalkyleneoxides, each of which may be straight or branched. Monoalkyl-capped polyalkyleneoxides, according to the invention, include polyethylene glycol ("PEG") homopolymers and polypropyleneglycol homopolymers. Other biocompatible polymers suitable for use, according to the invention, are dextran, polyvinylpyrrolidone and DL amino acids. In a preferred embodiment, the biocompatible polymer is end-capped at one end to help prevent cross derivatization. Suitable "capping" groups include straight or branched lower alkoxy groups; preferably, the monomethoxy group.

In one embodiment, the biocompatible polymer is activated prior to the coupling reaction, to make possible the formation of covalent bonds. According to this embodiment, a hydroxyl group, suitable for activation, is attached to the end of the bio-compatible polymer facing the agent. The invention provides several ways of activating the bio-compatible polymer, depending on the amino acid selected for covalent binding.

In a preferred embodiment, the bio-compatible polymer is a PEG homopolymer. The molecular weight of the PEG can be in the range of from about 300 up to about 60,000 daltons, more preferably between 1,000 and 20,000. The choice of molecular weight of a particular PEG can be made, taking into consideration the nature of the particular agent that the PEG will modify. One consideration in particular includes the number of amino groups or other functional groups of the agent that are available for modification. A particularly preferred PEG homopolymer of the present invention is monomethoxy polyethyleneglycol (mPEG) and derivatives thereof.

In a particular embodiment, the selected PEG can be mono methoxy succinimidyl butanoate (SBA)-PEG, succinimidyl carbonate (SC)-PEG, Aldehyde (ALD)-PEG, mPEG Succinimidyl succinate, mPEG succinimidyl succinamide, mPEG succinimidyl propionate SPA-(SPA-PEG), succinimidyl butanoate, succinimidyl carbonate of mPEG, succinimidyl ester of carboxymethylated mPEG, mPEG-oxycarbonylimidazole, mPEG nitrophenyl carbonates, mPEG trichlorophenyl carbonate, mPEG tresylate, mPEG maleic anhydride and mPEG methylmaleic anhydride, mixed anhydrides of mPEG succinate, mPEG acetic acid or mPEG propionic acid, mPEG aldehyde, mPEG maleimide, mPEG vinylsulfone and mPEG-orthopyridyldisulfide, and mPEG derivatives of phenylglyoxal.

The invention also provides for the use of the PEGs described in U.S. Patent Nos. 5,612,460; 5,808,096; 5,643,575; 5,672,662; 5,990,237; - all of which are hereby incorporated by reference.

In one aspect of the invention, there are provided PEG polymers corresponding to the formula:

Pₙ-L-A,

wherein (P) is a PEG; (n)=at least 1, 2 or 3; (L) is an linking moiety, covalently linked to each (P); and (A) represents an activating functional group capable of undergoing nucleophilic substitution. (A) can be, for example, a group capable of bonding with biologically active nucleophiles or moieties capable of doing the same. This polymer is capable of attaching to a polypeptide, among other types of agents.

The point of polymer attachment to a polypeptide, for example, depends upon the functional group (A). For example, (A) can be a succinimidyl succinate or carbonate that reacts with the epsilon amino groups oflysines. The PEG polymers also can be activated, using conventional chemistry, to link with any primary or secondary amino group, thiol group, carboxylic acid group, or reactive carbonyl group found on biologically-active materials. Other activating groups include those that are apparent to a skilled artisan.

The invention also contemplates the use of various linking moieties, which attach the PEG to an agent. In one embodiment, the linking moiety is an aliphatic group, comprising a chain of up to 18, and more preferably between 1-10, carbon atoms. A heteroatom, such as nitrogen, oxygen or sulfur may be included within the alkyl chain. The alkyl chain may also be branched at a carbon or nitrogen atom. In another aspect of the invention, (L) is a single nitrogen atom. In greater detail, suitable aliphatics include substituted alkyl diamines and triamines, lysine esters and malonic ester derivatives. The linking moieties, according to the invention, are preferably non-planar, so that the polymer chains are not rigidly fixed. The linking moiety (L) is also the means for attaching multiple PEGs, or "branches," to (A).

The linking moiety (L) and each (P) are preferably joined by a reaction between nucleophilic functional groups on both (P) and (L). Each (P) may be functionalized to undergo nucleophilic substitution and bond with (L). This functionalization process can easily be carried out according to conventional technology.

A wide variety of linkages are contemplated between (P) and (L). Urethane (carbamate) linkages are preferred, since urethane links are highly stable under a variety of physiological conditions, as reported by Larwood et al, Labeled Compounds Radiopharm., 21: 603-614 (1984). A carbamate bond between (P) and (L) can be formed, for example, by reacting an amino group, such as 1,3-diamino-2-propanol, with methoxypolyethylene glycol succinimidyl carbonate, as described in U.S. Pat. No. 5,122,614. Amide linkages are also contemplated linkages between (P) and (L), and can be formed by reacting an amino-terminated non-antigenic polymer, such as methoxy-polyethylene glycol-amine (mPEG amine), with an acyl chloride functional group.

Examples of other linkages between (P) and (L) include ether, amine, urea, and thio and thiol analogs thereof, as well as the thio and thiol analogs of the above-discussed urethane and amide linkages. These linkages are formed by methods well understood by those of ordinary skill in the art. Additional suitable linkages, as well as the formation thereof, can be determined by reference to U.S. Pat. No. 4,179,337.

The moiety (A) represents groups that "activate" the PEG polymers described herein and provide for the conjugation of these PEG polymers with one or more biologically active materials, for example, an agent or a therapeutic composition. Accordingly, (A) can be a moiety selected from:
I. Functional groups capable of reacting with an amino group, such as:
   a) carbonates, for example, p-nitrophenyl, or succinimidyl; b) carbonyl imidazole; c) azlactones; d) cyclic imide thiones; or e) isocyanates or isothiocyanates;
II. Functional groups capable of reacting with carboxylic acid groups and reactive carbonyl groups, including:
   a) primary amines; and b) hydrazine and hydrazide functional groups, such as acyl hydrazides, carbazates, semicarbamates, thiocarbazates;
III. Functional groups capable of reacting with mercapto or sulfhydryl groups, such as:
   phenyl glyoxals. This class of functional groups is disclosed, for example, in U.S. Pat. No.5,093,531; or
IV. Other nucleophiles capable of reacting with an electrophilic center, including hydroxyl, amino, carboxyl, thiol groups, and active methylene.

The (A) moiety also can include a "spacer" moiety located proximal to the aliphatic linking moiety, (L). The spacer moiety may be a heteroalkyl, alkoxy, alkyl containing up to, for example, 18 carbon atoms or even an additional polymer chain. These spacer moieties can be added by employing conventional synthesis techniques. Of course, the (A) moieties also are able to react with other moieties besides biologically active nucleophiles.

### Basic Methodologies

The basic inventive method entails administering a modified therapeutic agent or composition to a subject. Next, the blood of the subject is assayed for biological activity of the therapeutic. The subject is then treated with one or more doses of the modified agent or composition at levels useful in therapy. The blood of the subject is assayed again for biological activity of the therapeutic agent or composition. Any decrease in activity from the first measurement and subsequent measurements is ascertained. The foregoing may be repeated multiple times. The method also may be repeated with different lots of therapeutic agent, which differ in the extent of modification. An ideal level of modification is that which yields the smallest decrease in biological activity between doses.

The amount of modified agent or composition that is administered to a subject, as well as the methods of administration, can vary without departing from the scope of the invention. In general, the initial amount of modified therapeutic administered is an amount sufficient to allow for detection of the agent or composition's biological activity by a suitable assay. Preferably, the therapeutic is detectable at least about 12 to 48 hours after administration of the modified therapeutic.

After the initial treatment, the biological activity of the administered modified therapeutic in the blood, serum or other body fluid is assayed. A skilled artisan will be able to ascertain the appropriate period of administering the modified therapeutic and assaying the biological activity of the administered modified therapeutic. In one embodiment, the biological activity is assayed as early as about 30 minutes and as long as about 10 days after the dose; however, the time period between administering the therapeutic and assaying its biological activity may vary without departing from the scope of the invention. If biological activity is detected, one or more booster doses again may be administered to the subject in accordance with the methodology provided herein. After receiving this regimen of booster dosage(s), the subject may receive a third dose of modified agent at a concentration in an amount sufficient to assay the biological activity of the administered therapeutic, typically at a time of at least about 30 minutes to 10 days after the administration of this dosage. Thereafter, the biological activity of the administered therapeutics is measured by a suitable assay. This regimen - comprising (1) administering to a subject one or more booster doses of a modified agent or composition, and (2) administering an amount of modified agent at a concentration sufficient to allow for the detection of the agent's or composition's biological activity by a suitable assay, followed by (3) assaying the biological activity of the administered agent or composition - can be repeated additional times.

To provide meaningful comparative data, the invention contemplates repeating the foregoing methodology using the same therapeutic, but where the therapeutic is modified to different levels. The administration regimen, namely - administering to a subject (1) one or more booster doses of a modified agent, and thereafter (2) an amount of modified agentat a concentration in an amount sufficient to allow for the detection of the agent's biological activity by a suitable assay, followed by (3) assaying the biological activity of the administered agent - will reveal the amount of time the biological activity of this agent remains detectable in the subject to whom this modified agent was administered. Of course, the invention provides for the repetition of this process, wherein the agent of interest is modified to known, but different, percentages.

In another embodiment, the invention contemplates repeating the foregoing methodology, using two or more lots of the same therapeutic, but where the therapeutic is modified with a different modifying agent in each lot and wherein the therapeutic is modified to the same or substantially similar levels in each lot. Repeating the administration/assay methodology, essentially as described above, will reveal the modifying agents that are most conducive to prolonging the biological activity of the therapeutic that is administered to a subject. After a particular modifying agent has been selected, the invention also provides a regimen for determining an ideal extent of modification of the same therapeutic, using the selected modifying agent, such as the administration/assay regimen described above.

The process of discerning the modification conditions for a given agent often entails the selection of a particular percentage, or range thereof, among a group of agents, each of which have modified to a known, but different percentage, vis-à-vis each other. By comparing the data among these agents, which have been modified to different, but known, percentages, a skilled artisan will be able to deduce an ideal level for a particular agent. Typically, the methods of the invention result in increased circulating levels of the agents or compositions of the invention, thereby imparting an increased circulating half-life.

Determining a therapeutically effective amount is well within the purview of the skilled clinician and largely will depend on the exact identity of the agent or composition, the modifying agent, and the patient characteristics. General guidance can be found, for example, in the publications of the International Conference on Harmonisation. Such a determination specifically will depend on such factors as toxicity and efficacy profile of the inventive compound.

In an initial clinical trial, a patient in need of treatment or a normal volunteer typically is administered a modified agent or composition at a specified dose, usually low, at specified intervals for a period of time. In the absence of adverse effects, as determined by the clinician, this procedure may be repeated with successively higher doses of agent or composition. In this way potential toxic side-effects and parameters, such as bioavailability, may be determined using methods readily known in the art. Some typical pre-clinical and clinical parameters that are monitored are found in Remington's at chapters 27 and 28, pages 484-528.

With the results of the toxicology studies in mind, clinical trials for efficacy may be undertaken. For example, a patient being treated with a pharmaceutical agent will be administered a modified agent or composition. Via well-known methods, the circulating levels of the pharmaceutical agent or composition can be determined. Analysis of this circulating level as a function of experimental dose will allow the clinician easily to determine efficacious, nontoxic doses of the modified agent or composition as relevant to any pharmaceutical agent or composition.

Although the foregoing detailed description and the following examples set forth representative preferred embodiments of the invention, they are not intended as exclusive embodiments. In view of the material presented, one of ordinary skill in the art readily would appreciate further embodiments that fall within the scope of the invention.

The following examples are provided to illustrate the invention.

### EXAMPLES

### Example 1:

### Determination of Extent of PEGylation Using the Fluorescamine Assay

The fluorescamine assay determines the extent of PEGylation of *Pseudomonas 7A* Glutaminase-Asparaginase (PGA). Fluorescamine reacts with unmodified lysine groups to give a fluorescent derivative. To perform the assay, the protein sample (either unmodified PGA or PEG-modified) was diluted to make a solution of 200 µg/ml (approximately 2 ml of solution). The solution was added in portion to a set of five test tubes: 0.1 ml in the first, 0.2 ml in the second, et cetera. Each tube was diluted to 2.0 ml with 0.1 M sodium phosphate buffer, pH 8.0. A sixth tube containing only 2.0 ml buffer was included as a blank. One ml of fluorescamine solution (0.3 mg/ml in acetone) was added to the sample during vigorous vortexing. After 5 minutes the sample was assayed on a fluorescence spectrophotometer with excitation set at 390 nm and emission set at 475 nm.

### Example 2:

### Assay to Detect Inactivating Antibody Development to PEGylated Pseudomonas 7A Glutaminase-Asparaginase (PGA)

Using a model that closely resembles therapeutic conditions, we developed a method of effective PEGylation of the protein *Pseudomonas 7A* Glutaminase-Asparaginase (PGA). PEGylation protects or shields the therapeutic enzyme from the immune system and, therefore, from inactivation. The degree ofPEGylation is critical. If too little, the protein is not covered and antibody-mediated inactivation can still occur. Too much PEGylation can disrupt the tertiary structure of the protein, thus exposing new antigenic determinants to the immune system. The following is a description of the method that we use to test effective PEGylation of PGA.

Mice were injected with an initial dose of PGA and bled after eighteen hours by the method of retro-orbital bleeding. The glutaminase assay was performed on the serum following the first injection ofPEGylated PGA to determine the activity of PGA recovered in the serum. Following the initial injection, booster injections of a significantly lower dose were given every other day. Following each week, another injection with dosage suitable to perform the glutaminase assay was given and the mice were bled after 18 hours to collect serum so that the assay may be repeated as described above. When no activity was detected in the serum, the experiment was terminated. Without PEGylation, antibodies against PGA will develop within eight days and PGA will be inactivated in the blood in a few hours, as opposed to its over 48 hr half-life in naïve mice.

### Example 3:

### Enzyme linked immunosorbent assay:

Other methods to determine the extent of antibody formation have proven unable to predict the effectiveness of PEGylation in protecting the therapeutic protein during prolonged periods of therapy. An enzyme-linked immunosorbent assay (ELISA) was performed to determine the extent of Anti- *Pseudomonas 7A* Glutaminase-Asparaginase (PGA) antibody present in the serum. Plates were coated with a 10 µg/ml solution of PGA. Serum isolated from mice bled eighteen hours following an injection of various PEGylated forms of PGA after two weeks of treatment were then added to the wells. Anti-Mouse IgG +IgM conjugated to alkaline phosphatase was added as a secondary antibody. A colorimetric assay was then performed to determine the level of anti-PGA antibodies in the serum of treated mice. ELISA results showed that despite detectable levels of antibody in the serum, some preparations of PEG-PGA (SBA-PGA) were able to maintain enzyme activity in the serum at the usual half-life levels. However, some ALD-PGA preparations showed no detectable antibody development on ELISA, even though the enzyme had been successfully cleared from the serum, as indicated in the following table.

**TABLE 1**

| a. Sample | **Avg. A₄₀₅ Reading (ELISA)** | b. % Mice with Enzyme Activity |
|---|---|---|
| | | **using the in vivo method** |
| Blank | 0.096 | NA |
| PGA Positive Control | 1.250 | 0 % |
| SPA-PGA Prep. 1 | 0.202 | 100 % |
| SBA-PGA Prep. 1 | 0.700 | 100 % |
| SBA-PGA Prep. 2 | 0.882 | 80 % |
| SBA-PGA Prep. 3 | 0.812 | 25 % |
| SBA-PGA Prep. 4 | 1.019 | 20 % |
| ALD-PGA Prep. 3 | 0.095 | 100 % |
| ALD-PGA Prep. 4 | 0.094 | 40 % |

Therefore, ELISA is not predictive of clearance of activity, and the glutaminase assay (or relevant biological activity for other agents) must be performed on serum to determine the success of different PEGylation strategies.

### Example 4

### Optimization of PEGylation Using Succinimidyl Succinate (SS) PEG:

The N-hydroxysuccinimidyl (or NHS) active ester of PEG succinate (SS-PEG) is commonly used for attachment of PEG to proteins or peptides. This derivative reacts with amino groups on proteins under mild conditions within short periods of time (approximately 30 minutes, pH 8.5), yielding extensively modified yet active conjugates. A potential problem with this derivative is that the ester linkage in the backbone is susceptible to hydrolytic cleavage. SS-PE G has the following structure:

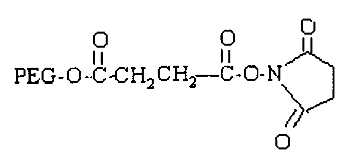

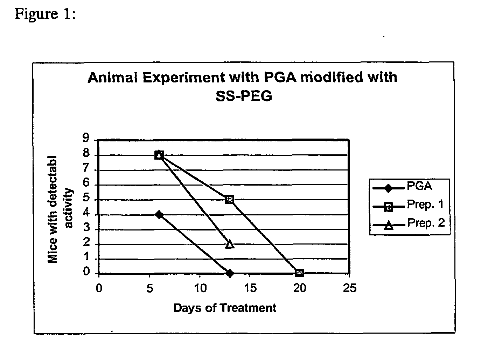

PEGylation with SS-PEG was achieved by dissolving the purified PGA protein in 50 mM Sodium phosphate buffer pH 8.0 to a concentration of 8.0 mg/ml. Two preparations of SS-PEG were made: Preparation 1: 10 mg of SS-PEG 5000 added to 200 µl of 8.0 mg/ml solution of PGA and Preparation 2: 20 mg /200 µl. These preparations along with unmodified PGA were evaluated in mice according to the procedure in example 2 for stability during prolonged periods of therapy. After six days of therapy, PGA activity could be recovered from animals treated with unmodified PGA as well as preparations 1 and 2. Activity could not be recovered from animals treated with unmodified PGA or preparation 2 after two weeks of therapy. No activity remained in the serum of animals treated with any preparation after three weeks of therapy. The results are summarized in Figure 1. These findings are most likely due to the hydrolytic cleavage of the SS-PEG backbone as mentioned above.

### Example 5

### Optimization of PEGylation Using Benzotriazole carbonate (BTC) PEG 5000

While not as reactive as some of the N-hydroxysuccinimidyl (NHS) active esters, BTC-PEG is an efficient modifier of peptide and protein amino groups. BTC-PEG is sufficiently reactive to produce extensively modified PEG-proteins under mild conditions within short periods of time. The BTC-PEG has the following structure:

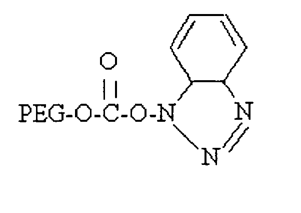

PEGylation with BTC-PEG was achieved by dissolving the purified PGA protein in 50 mM Sodium phosphate buffer to a concentration of 8.0 mg/ml. PEGylation was performed using sodium phosphate buffer at pH 6.5 and pH 8.0 with similar results. PEG was added in amounts ranging from 2.5 mg /200 µl PGA solution to 20 mg/200 µl. The PEGylation reaction was conducted by tumbling the reaction mix at room temperature for 60 minutes. The unbound PEG was removed by dialysis against 50 mM sodium phosphate buffer pH 7.5. PGA PEGylated with BTC-PEG at 5 mg/200 µl PGA solution yielded 16.5% PEGylation and at 10 mg/200µl yielded 26% PEGylation. PEGylation at 15 and 20 mg/200 µl gave 57 and 65% PEGylation, respectively. However, PEGylation at higher concentrations resulted in the loss of approximately 75% enzyme activity.

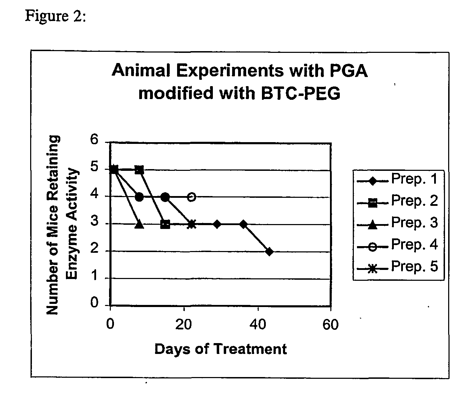

BTC-PEG was successful at extending the half-life of PGA in the serum. Success was not seen in delaying inactivating antibody development by the host to PGA (Figure 2). In all concentrations of PEG tested, inactivating antibody development occurred after only two weeks of treatment at both PEGylation pH values (6.5, 8.0).

### Example 6

### Optimization of PEGyation Using Aldehyde (ALD) PEG

The PEG bearing the aldehyde group undergoes a reductive amination reaction with primary amines in the presence of sodium cyanoborohydride. Unlike other electrophilically activated groups, the aldehyde reacts only with amines. Proteins modified in this fashion retain amine groups and associated charge in solution, which can be important for maintaining protein conformation and activity. Additionally, there is no linker between PEG and protein to act as an antigenic site or lead to toxicity upon breakdown. This PEG is commercially available from Shearwater Polymers, Inc. and has the following structure: 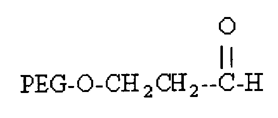 PEGylation using ALD-PEG is performed by dissolving purified lyophilized PGA in 50 mM Sodium Phosphate pH 6.5 to a concentration of 8.0 mg/ml. ALD-PEG 2,000 was then added at the following concentrations: Prep. 1: 2.5 mg/ 200 µl, Prep. 2: 7.5 mg/200 µl, Prep. 3: 10 mg/200 µl, and Prep. 4: 12.5 mg/200 µl. Sodium cyanoborohydride (31.5 mg/ml) was then added at 50 µl per ml. The PEG was dissolved and the solution tumbled at room temperature for 18 to 24 hours. Preparation 1 was PEGylated at 31 %. Preparation 2 was PEGylated at 51 %. Preparation 3 was PEGylated at 66%. Preparation 4 was PEGylated at 74%. None of the ALD-PEG preparations resulted in significant loss of enzyme activity.

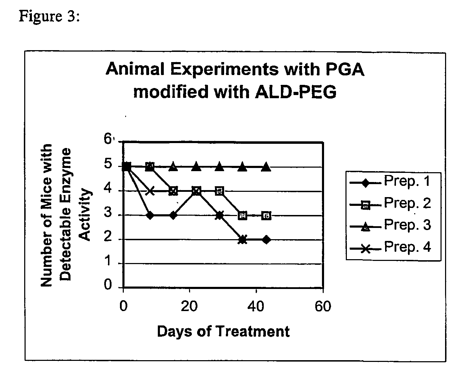

Preparations 2 and 3 proved to be the best suited for long term therapy. Following one month of treatment, all five mice given preparation 3 and four of five mice given preparation 2 retained enzyme activity 18 hours following an injection. The results indicate that the percent PEGylation of PGA needs to fall within a range of 45-65% when using ALD-PEG2000. These results are shown in Figure 3. PEGylation at lower or higher amounts, as in preparations 1 and 4 respectively, resulted in inactivation of the enzyme in the serum during the course of long-term therapy. PEGylation using aldehyde PEG is superior to PEGylation using other activated PEGs because there is less activity loss, it is effective over a broad PEGylation range, and only lysine groups are modified.

### Example 7

### Optimization of PEGylation Using Succinimidyl Carbonate (SC) PEG

Extensive experimentation on the PEGylation of *Pseudomonas 7A* Glutaminase-Asparaginase (PGA) was performed using SC-PEG commercially available from Shearwater Polymers, Inc., as well as, that produced according to the procedure described by Miron and Wilchek, 1993 (Miron,T and M. Wilchek., 1993. Bioconjugate Chemistry, 4:568-569). This PEG is relatively unreactive, but still an efficient modifier of proteins. Biological activities of such conjugates are usually well preserved. The reaction between SC-PEG and amino-containing molecules results in formation of a urethane linkage, which is stable to hydrolysis. SC-PEG has the following structure:

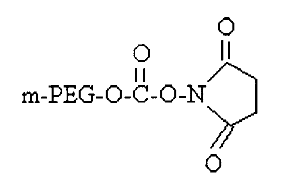

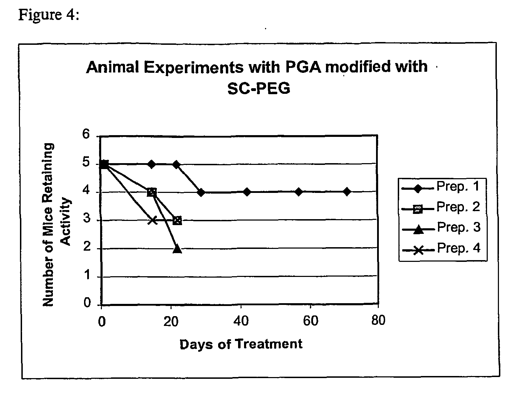

The PEGylation procedure consisted of dissolving the purified protein in 50 mM Sodium Bicarbonate buffer pH 8.8 to a concentration of approximately 8.0 mg/ml. Four preparations of SC-PGA were made in the following manner. To a 200 µl solution of the 8.0 mg/ml solution, 2.5 mg SC-PEG were added in prep. 1, 5 mg in prep. 2, 7.5 mg in prep. 3, and 10 mg in prep.4. Preparation 1 resulted in 31 % PEGylation with an enzyme loss of 30%. Preparation 2 resulted in 49% PEGylation and 41% activity loss. Preparation 3 resulted in 55% PEGylation and 44% activity loss. Preparation 4 resulted in 61 % PEGylation with 56% activity loss. All mice receiving Preparation 1 of the SC-PGA retained circulating PGA activity at 18 hours post-injection after three weeks and four of five mice still had circulating PGA activity at 18 hours post injection after 10 weeks of therapy. Despite the higher percentages of PEGylation, preparations 2, 3 and 4 were not effective in maintaining circulating enzyme activity 18 hours post-injection in several of the mice by the third week of treatment. These results are shown in Figure 4. Thus 31 % is the optimum extent of modification of PGA, using SC-PEG5000.

### Example 8

### Use of Mono Methoxy Succinimidyl Butanoate Poly(Ethylene Glycol)(SBA-PEG 5000) to Prevent Inactivating Antibody Formation to Pseudomonas 7A Glutaminase-Asparaginase (PGA)

We have used an N-hydroxysuccinimidyl ester of monomethoxy poly(ethylene glycol) butanoic acid, MW5000 (SBA-PEG 5000) to modify Pseudomonas 7A Glutaminase-Asparaginase (PGA). SBA-PEG has the following structure:

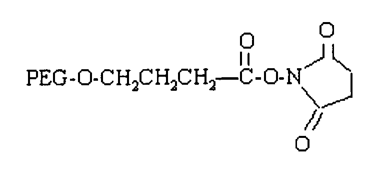

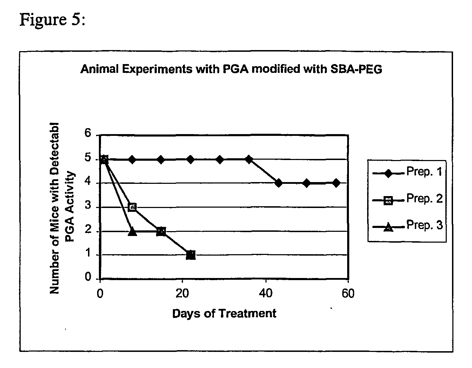

The reaction between lysine, terminal amines, and the active esters produces a stable amide linkage. The lack of an ester linkage in the backbone of the PEG provides increased stability. This PEG is commercially available through Shearwater Polymers, Inc. PGA was PEGylated with SBA in several ratios to determine an ideal ratio. The PEGylation procedure consisted of dissolving the purified protein in 50 mM Sodium Bicarbonate buffer pH 9.5 to a concentration of approximately 8.0 mg/ml. The SBA-PEG was added in the following amounts: Preparation 1: 3 mg/200 µl; Preparation 2: 5 mg/200 µl; and Preparation 3: 10 mg/200 µl. The reaction mixture was tumbled at room temperature for 90 minutes. The PEGylated PGA was then dialyzed against 50 mM Sodium Phosphate pH 7.5. PEGylation resulted in little loss in enzyme activity even at the highest ratio of PEG used, where significant activity loss is often observed when using other PEGs. Preparation 1 resulted in 35%, Prep. 2 in 58%, and Prep. 3 in 71 % PEGylation according to the fluorescamine assay described in Example 1. Glutaminase was not inactivated in the serum of the animals treated according to example 2, using Preparations 2 and 3 for two weeks of therapy; however, when Preparation 1 was used, serum glutaminase activity was high, even after eight weeks of therapy. Thus 35% PEGylation of PGA using mSBA-PEG5000 is optimum for protecting the protein during long-term therapy. The results are summarized in Figure 5.

### Example 9

### Optimization of PEGylation Using Succinimidyl Propionate (SPA) PEG

Similar to SBA-PEG, SPA-PEG is very stable due to its lack of an ester linkage in the backbone of the PEG. The reactivity to protein of the two compounds is very similar with SPA being slightly more reactive. SPA-PEG has the structure depicted below:

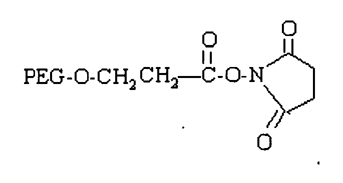

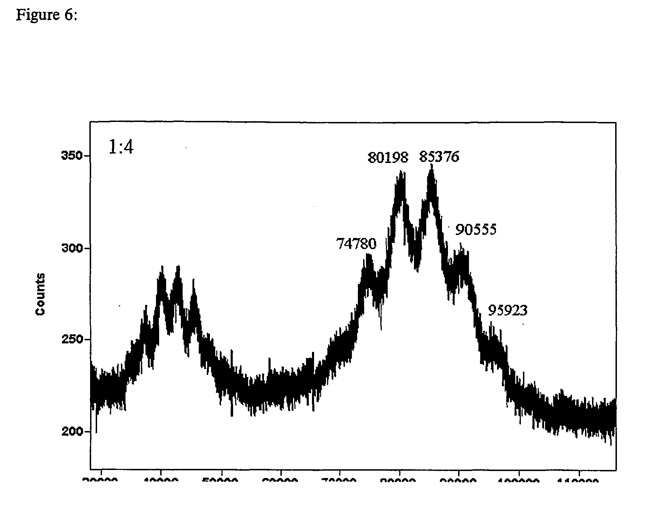

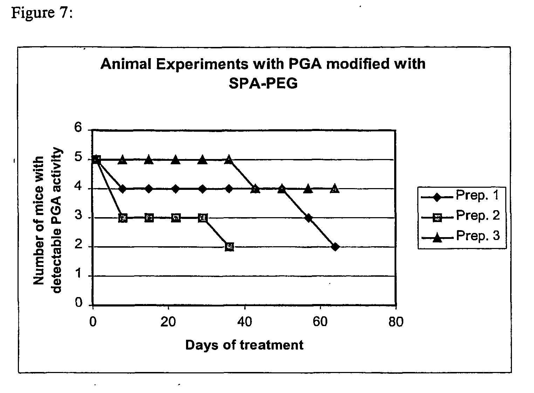

PEGylation using SPA-PEG was performed similarly to SBA-PEG by dissolving the purified protein in 50 mM Sodium Bicarbonate buffer pH 9.5 to a concentration of approximately 10 mg/ml. The SPA-PEG was then added in the following amounts: Preparation 1: 2 mg/200 µl; Preparation 2: 3 mg/200 µl; and Preparation 3: 4 mg/200 µl. The reaction mixture was tumbled at room temperature for 60 minutes. The PEGylated PGA was then dialyzed against 50 mM Sodium Phosphate pH 7.5. Preparation 1 was PEGylated to about 40%, Preparation 2 to about 50% and Preparation 3 to about 55 %. Preparation 3 was effective in retaining circulating PGA activity 18 hours post-injection even after prolonged periods of therapy. Matrix-assisted-laser-desorption-ionization mass spectrometry (MALDI) analysis of preparation 3 revealed that the molecular weight of PEGylated PGA was between 70,000 and 110,000 daltons per monomer (Figure 6). Preparation 3 was found to be PEGylated with 4 out of 5 animals maintaining high circulating enzyme activity even after 60 days of therapy (Figure 7)

### Example 10

### Formulation of PEGylated PGA

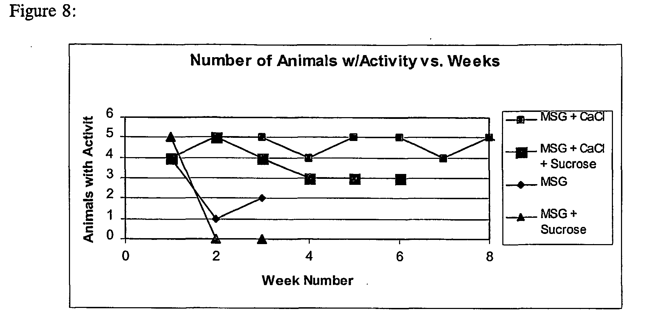

Lyophilization of PEGylated PGA was found to significantly affect the activity of the enzyme and disrupt the ability of PEGylation to prevent inactivating antibody formation. A variety of compounds were tested to observe which compounds were capable of conferring additional stability to PGA against the effects of heating. The compounds that were most successful (CaCl₂, sodium glutamate, and sucrose) were then tested for their ability to serve as protective agents for PEGylated PGA during lyophilization. Previously, lyophilization was found to result in a decrease in activity of approximately 50% and to lessen the ability of PEGylation to prevent inactivating antibody formation. SBA-PGA 1:3 was lyophilized in 5 mM potassium phosphate buffer using 10 mM sucrose, 10 mM sodium glutamate and a combination using 10 mM of both compounds. No significant decrease in activity was found. No enzyme activity was recovered 18 hours post-injection in animals treated for two weeks with SBA-PGA lyophilized in the presence of 5mM potassium phosphate by itself or in combination with 10 mM sucrose. However, glutamate at 10 mM was found to be effective in protecting SBA-PGA during lyophilization with good circulating PGA activity 18 hours post-injection even after 10 weeks of therapy (Figure 8).

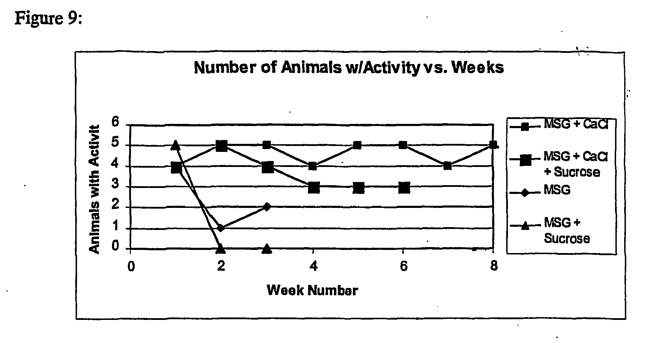

Further experiments were performed to determine the excipients that best protect PGA PEGylated with SPA-PEG 5000 during lyophilization. The following excipients were tested: (i) 10 mM MSG alone, (ii) 10 mM MSG + 5mM CaCl₂, (iii) 10 mM MSG + 10% Sucrose, (iv) 10 mM MSG + 5mM CaCl₂ + 10% Sucrose. PEG-PGA with the above excipients was lyophilized as follows: step 1 - shelf freeze at -20°C for 2 hours, step 2 - primary drying at -20°C for 4 hours, step 3 - secondary drying at 4°C for 12 hours, and step 4 - tertiary drying at 20°C for 3 hours. The lyophilized proteins were stored at 4°C in a dessicator until further use. The four preparations were tested in animals to study the protection afforded by the excipients during lyophilization to PGA PEGylated with SPA-PEG 5000 according to the method described in example 2. As seen in Figure 9, 10 mM MSG + 5mM CaCl₂ was the best excipient combination for stabilizing PGA PEGylated with SPA-PEG 5000 during lyophilization. These excipients were used in our scale-up studies and for producing PEG-PGA for animal toxicology and human clinical testing.

## Claims

1. A method for determining the type of biocompatible polymer, the extent of modification, and the conditions for modification of a therapeutic agent with a biocompatible polymer to prevent host-mediated inactivation of said therapeutic agent when covalently modified by said biocompatible polymer, comprising:
(a) assaying a biological activity of a first modified therapeutic agent after said first modified therapeutic agent has been administered to a non-human subject, wherein said first modified therapeutic agent is covalently modified with a biocompatible polymer;
(b) assaying the biological activity of said first modified therapeutic agent after at least one booster dose of said first modified therapeutic agent has been administered to said non-human subject;
(c) assaying the biological activity of a second modified therapeutic agent after said second modified therapeutic agent has been administered to a non-human subject, wherein said second modified therapeutic agent is covalently modified with a biocompatible polymer and wherein at least one condition selected from the group consisting of the type of biocompatible polymer, the extent of modification, and the conditions for modification differs from the conditions of said first modified therapeutic agent;
(d) assaying the biological activity of said second modified therapeutic agent after at least one booster dose of said second modified therapeutic agent has been administered to said non-human subject; and
(e) comparing the biological activity of said first modified therapeutic agent with the biological activity of said second modified therapeutic agent to select the type of biocompatible polymer, the extent of modification, and the conditions for modification that prevent host-mediated inactivation of said therapeutic agent when covalently modified by said biocompatible polymer,
wherein said therapeutic agent comprises an enzyme and said biological activity comprises an enzyme catalyzing a reaction.

2. The method of claim 1, wherein said second modified therapeutic agent is modified with the same biocompatible polymer as said first modified therapeutic agent.

3. The method of claim 1, wherein said biocompatible polymer is polyethylene glycol (PEG).

4. The method of claim 3, wherein said PEG is selected from the group consisting of monomethoxy succinimidyl butanoate (SBA)-PEG, succinimidyl carbonate (SC)-PEG, aldehyde (ALD)-PEG, and succinimidyl propionate (SPA)-PEG.

5. The method of claim 1, wherein said second modified therapeutic agent and said first modified therapeutic agent are modified with different biocompatible polymers.

6. The method of claim 1, wherein said enzyme is glutaminase-asparaginase.

7. A method of preparing a pharmaceutical composition where host-mediated inactivation is prevented, comprising selecting the type of biocompatible polymer, the extent of modification, and the conditions for modification of a therapeutic agent by the method of claim 1 and modifying said therapeutic agent according to the type of biocompatible polymer, the extent of modification, and the conditions for modification selected, wherein said therapeutic agent comprises an enzyme.

8. The method of claim 7, wherein said pharmaceutical composition further comprises an excipient.

9. The method of claim 7, wherein said therapeutic agent comprises glutaminase-asparaginase.

10. The method of claim 7, wherein said therapeutic agent is a *Pseudomonas* glutaminase-asparaginase modified with polyethylene glycol.

11. The method of claim 7, wherein said therapeutic agent is modified to an extent of from about 21 % to about 49% by SC-PEG 5000.

12. The method of claim 7, wherein said therapeutic agent is modified from about 30% to about 40% by SBA-PEG 5000.

13. The method of claim 7, wherein said therapeutic agent is modified to an extent of from about 45% to about 65% by ALD-PEG 2000.

14. The method of claim 7, wherein said therapeutic agent is modified to an extent of from about 25% to about 65% by SPA-PEG 5000.

## Patentansprüche

1. Verfahren zur Bestimmung des Typs eines biokompatiblen Polymers, des Ausmaßes der Modifikation und der Bedingungen für die Modifikation eines Therapeutikums mithilfe eines biokompatiblen Polymers zur Verhinderung einer wirtsvermittelten Inaktivierung des Therapeutikums, wenn dieses kovalent von dem biokompatiblen Polymer modifiziert wird, umfassend
(a) Untersuchen einer biologischen Aktivität eines ersten modifizierten Therapeutikums nach der Verabreichung des ersten modifizierten Therapeutikums an einen nicht humanen Patienten, wobei das erste modifizierte Therapeutikum kovalent mithilfe eines biokompatiblen Polymers modifiziert ist;
(b) Untersuchen der biologischen Aktivität des ersten modifizierten Therapeutikums nach der Verabreichung mindestens einer Verstärkungsdosis des ersten modifizierten Therapeutikums an den nicht humanen Patienten;
(c) Untersuchen der biologischen Aktivität eines zweiten modifizierten Therapeutikums nach der Verabreichung des zweiten modifizierten Therapeutikums an einen nicht humanen Patienten, wobei das zweite modifizierte Therapeutikum kovalent mithilfe eines biokompatiblen Polymers modifiziert ist und wobei sich mindestens eine Bedingung, ausgewählt aus der Gruppe, bestehend aus Typ des biokompatiblen Polymers, Ausmaß der Modifikation und Bedingungen der Modifikation, von den Bedingungen des ersten modifizierten Therapeutikums unterscheiden;
(d) Untersuchen der biologischen Aktivität des zweiten modifizierten Therapeutikums nach der Verabreichung mindestens einer Verstärkungsdosis des ersten modifizierten Therapeutikums an den nicht humanen Patienten; und
(e) Vergleich der biologischen Aktivität des ersten modifizierten Therapeutikums mit der biologischen Aktivität des zweiten modifizierten Therapeutikums zur Auswahl des Typs des biokompatiblen Polymer, des Ausmaßes der Modifikation und der Bedingungen für die Modifikation zur Verhinderung einer wirtsvermittelten Inaktivierung des Therapeutikums, wenn dieses kovalent von dem biokompatiblen Polymer modifiziert wird,
wobei das Therapeutikum ein Enzym umfasst und die biologische Aktivität ein Enzym das eine Reaktion katalysiert umfasst.

2. Verfahren nach Anspruch 1, wobei das zweite modifizierte Therapeutikum mithilfe desselben biokompatiblen Polymers modifiziert ist wie das erste modifizierte Therapeutikum.

3. Verfahren nach Anspruch 1, wobei das biokompatible Polymer Polyethylenglykol (PEG) ist.

4. Verfahren nach Anspruch 3, wobei das PEG ausgewählt ist aus der Gruppe, bestehend aus Monomethoxysuccinimidylbutanoat (SBA)-PEG, Succinimidylcarbonat (SC)-PEG, Aldehyd (ALD)-PEG und Succinimidylpropionat (SPA)-PEG.

5. Verfahren nach Anspruch 1, wobei das zweite modifizierte Therapeutikum und das erste modifizierte Therapeutikum mithilfe verschiedener biokompatibler Polymere modifiziert sind.

6. Verfahren nach Anspruch 1, wobei das Enzym Glutaminaseasparaginase ist.

7. Verfahren zur Herstellung einer pharmazeutischen Zusammensetzung, wobei die wirtsvermittelte Inaktivierung verhindert ist, umfassend das Auswählen des Typs eines biokompatiblen Polymers, des Ausmaßes der Modifikation und der Bedingungen der Modifikation eines Therapeutikums mittels des Verfahrens nach Anspruch 1 und Modifizieren des Therapeutikums in Übereinstimmung mit dem Typ des biokompatiblen Polymers, dem Ausmaß der Modifikation und den ausgewählten Bedingungen der Modifikation, wobei das Therapeutikum ein Enzym umfasst.

8. Verfahren nach Anspruch 7, wobei die pharmazeutische Zusammensetzung weiterhin ein Hilfsmittel umfasst.

9. Verfahren nach Anspruch 7, wobei das Therapeutikum Glutaminaseasparaginase umfasst.

10. Verfahren nach Anspruch 7, wobei das Therapeutikum eine mit Polyethylenglycol modifizierte *Pseudomonas* Glutaminaseasparaginase ist.

11. Verfahren nach Anspruch 7, wobei das Therapeutikum in einem Ausmaß von etwa 21 % bis etwa 49 % durch SC-PEG 5000 modifiziert ist.

12. Verfahren nach Anspruch 7, wobei das Therapeutikum in einem Ausmaß von etwa 30 % bis etwa 40 % durch SBA-PEG 5000 modifiziert ist.

13. Verfahren nach Anspruch 7, wobei das Therapeutikum in einem Ausmaß von etwa 45 % bis etwa 65 % durch ALD-PEG 2000 modifiziert ist.

14. Verfahren nach Anspruch 7, wobei das Therapeutikum in einem Ausmaß von etwa 25 % bis etwa 65 % durch SPA-PEG 5000 modifiziert ist.

## Revendications

1. Méthode permettant de déterminer le type de polymère biocompatible, l'étendue de la modification et les conditions de modification d'un agent thérapeutique par un polymère biocompatible afin d'empêcher l'inactivation, induite par l'hôte dudit agent thérapeutique lorsqu'il subit une modification covalente par ledit polymère biocompatible, comprenant :
(a) le dosage d'une activité biologique d'un premier agent thérapeutique modifié après avoir administré à un sujet non humain ledit premier agent thérapeutique modifié, où ledit premier agent thérapeutique a été modifié par une modification covalente par un polymère biocompatible ;
(b) le dosage de l'activité biologique dudit premier agent thérapeutique modifié après avoir administré audit sujet non humain au moins une dose de rappel dudit premier agent thérapeutique modifié ;
(c) le dosage de l'activité biologique d'un second agent thérapeutique modifié après avoir administré à un sujet non humain ledit second agent thérapeutique modifié, où ledit second agent thérapeutique a été modifié par une modification covalente par un polymère biocompatible et où au moins une condition sélectionnée dans le groupe constitué du type de polymère biocompatible, de l'étendue de la modification et des conditions de modification diffère des conditions dudit premier agent thérapeutique modifié ;
(d) le dosage de l'activité biologique dudit second agent thérapeutique modifié après avoir administré audit sujet non humain au moins une dose de rappel dudit second agent thérapeutique modifié ; et
(e) la comparaison de l'activité biologique dudit premier agent thérapeutique modifié avec l'activité biologique dudit second agent thérapeutique modifié dans le but de sélectionner le type de polymère biocompatible, l'étendue de la modification et les conditions de modification qui empêchent l'inactivation induite par l'hôte dudit agent thérapeutique lorsqu'il subit une modification covalente par ledit polymère biocompatible,
où ledit agent thérapeutique comprend une enzyme et ladite activité biologique comprend une enzyme qui catalyse une réaction.

2. La méthode de la revendication 1, où ledit second agent thérapeutique modifié est modifié par le même polymère biocompatible que ledit premier agent thérapeutique modifié.

3. La méthode de la revendication 1, où ledit polymère biocompatible est le polyéthylène glycol (PEG).

4. La méthode de la revendication 3, où ledit PEG est sélectionné dans le groupe constitué du monométhoxy butanoate de succinimidyle (SBA)-PEG, du carbonate de succinimidyle (SC)-PEG, de l'aldéhyde (ALD)-PEG et du propionate de succinimidyle (SPA)-PEG.

5. La méthode de la revendication 1, où ledit second agent thérapeutique modifié et ledit premier agent thérapeutique modifié sont modifiés par des polymères biocompatibles différents.

6. La méthode de la revendication 1, où ladite enzyme est la glutaminase-asparaginase.

7. Méthode de préparation d'une composition pharmaceutique dans laquelle l'inactivation induite par l'hôte est empêchée, comprenant la sélection du type de polymère biocompatible, de l'étendue de la modification et des conditions de modification d'un agent thérapeutique à l'aide de la méthode de la revendication 1 et la modification dudit agent thérapeutique en fonction du type de polymère biocompatible, de l'étendue de la modification et des conditions de modification sélectionnés, où ledit agent thérapeutique comprend une enzyme.

8. La méthode de la revendication 7, où ladite composition pharmaceutique comprend également un excipient.

9. La méthode de la revendication 7, où ledit agent thérapeutique comprend de la glutaminase-asparaginase.

10. La méthode de la revendication 7, où ledit agent thérapeutique est une glutaminase-asparaginase de *Pseudomonas* modifiée par le polyéthylène glycol.

11. La méthode de la revendication 7, où ledit agent thérapeutique est modifié dans des proportions d'environ 21 % à environ 49 % par le SC-PEG 5000.

12. La méthode de la revendication 7, où ledit agent thérapeutique est modifié dans des proportions d'environ 30 % à environ 40 % par le SBA-PEG 5000.

13. La méthode de la revendication 7, où ledit agent thérapeutique est modifié dans des proportions d'environ 45 % à environ 65 % par l'ALD-PEG 2000.

14. La méthode de la revendication 7, où ledit agent thérapeutique est modifié dans des proportions d'environ 25 % à environ 65 % par le SPA-PEG 5000.
